# EUROPEAN PATENT APPLICATION

(11) **EP 2 045 601 A1**
(43) Date of publication of application: **08.04.2009**
(21) Application number: 07104924.1
(22) Date of filing: 26.03.2007
(51) Int. Cl.: G01N 33/50, G01N 33/53, G01N 33/543, G01N 33/545, G01N 33/569

(54) **Use of microcarrier beads for detection and/or isolation of cells by flow cytometry and/or dielectrophoresis**

(71) Applicant: Koninklijke Philips Electronics N.V., 5621 BA Eindhoven (NL)
(72) Inventor: The designation of the inventor has not yet been filed
(74) Representative: Golla-Franz, Anke Lucia

(57) **Abstract**

The present invention pertains to methods of and devices for detecting and/or isolating cells. Cells are allowed to associate to microcarrier beads directly or through binding to probes displayed on the microcarrier beads and subsequently subjected to flow cytometry and/or dielectrophoresis.

## Description

### SUBJECT OF THE INVENTION

The present invention pertains to methods of and devices for detecting and/or isolating cells by optical means including flow cytometry and/or dielectrophoresis. To this end, the methods and devices in accordance with the invention make use of microcarrier beads.

### BACKGROUND OF THE INVENTION

Rapid cell counting is typically performed by relying on flow cytometry, with fluorescence activated cell sorting (FACS) being representative for this type of methodology.

These modem flow cytometry techniques have in common that they count cells on an individual basis. Typically, a suspension of cells is hydrodynamically focussed in a narrow flow stream that passes through a detection chamber at high speed. A typical nozzle size for the focusing stream in a flow cytometer is in the order or 70 to 100 µm. If such a nozzle is coupled to high-speed fluidic flow, it is ensured that cells pass through the detection volume in a single file and are counted one by one. Modem flow cytometers typically reach detection speeds of 10,000 to 40,000 cells/sec.

However, flow cytometry suffers from the drawback that it usually cannot be used to analyse multi-cell aggregates such as multi-spheroids. The reason is that multi-cellular aggregates are typically of too large size to efficiently pass the nozzle of a flow cytometer and frequently lead to problems such as instrument clogging. On the other side, there is an interest in analysing multi-cellular assemblies as they more correctly represent the events that occur, e.g. within a cellular tumour.

Another method for detecting cells is dielectrophoresis. Dielectrophoresis relies on the application of a non-uniform electrical field to samples that are polarisable. When a polarisable electrically uncharged particle such as a cell is placed in such an inhomogeneous electrical field, it will become polarised and experience a net driving form due to the non-uniformity of the electrical field. This force can either be in the direction of increasing field strength (so-called positive DEP) or decreasing field strength (negative DEP). The point at which the electrical field changes the force on a cell from positive to negative DEP (and visa versa) is called the cross-over frequency, and this cross-over frequency can be used to discriminate various cell types. However, use of DEP for isolating and detecting specific cells from complex samples can be hampered by the fact that various cell types may not differ with respect to their polarisation characteristics sufficiently enough to allow separation based on DEP.

In view of this situation there is a continuing need in the art for further methods of and devices for isolating and detecting cells and particularly multi-cellular samples in an efficient and straightforward way.

### OBJECT AND SUMMARY OF THE INVENTION

It is one objective of the present invention to provide methods for isolating and/or detecting single cells as well as multi-cellular assemblies from different cellular samples such as e.g. blood.

It is a further objective of the present invention to provide devices that allow efficient detection and/or isolation of single cells and/or multi-cellular assemblies in cellular samples such as e.g. blood.

These objectives as well as others which will become apparent from the ensuing description are attained by the subject matter of the independent claims. Some of the more specific embodiments of the present invention are defined by the dependent claims.

The present invention in one embodiment relates to a method of detecting and/or isolating at least one cell comprising the steps of
- providing at least one microcarrier bead;
- contacting said at least one microcarrier bead with at least one cell such that said at least one cell associates with said at least one microcarrier bead;
- detecting and/or isolating said cell-associated microcarrier bead.

Said cell associated-microcarrier beads may be detected by optical detection methods, magnetic detection methods and/or dielectrophoresis.

The optical detection methods include optical imaging, image processing approaches and preferably flow cytometry.

Typically said microcarrier beads have a diameter of about 20 to about 1000 µm. A preferred diameter can be in the range of about 75 µm to about 350 µm. A size of about 100 µm to about 200 µm and about 150 µm can also be preferred.

The microcarrier beads comprise bead materials that render them suitable for application in optical detection devices such as flow cytometers and/or dielectrophoresis devices. Said microcarrier beads may thus be made from a material that is polarizable and can experience a force in an inhomogeneous electrical field. In this case, the beads can be moved by dielectrophoresis.

In general, said microcarrier beads will comprise bead materials selected from the group comprising polystyrene and poly-lactide.

The method in accordance with the invention may be performed by contacting the microcarrier beads with cells in cell culture such that the cells associate with the microcarrier beads during growth.

In one of the preferred embodiments of the invention, the microcarrier beads may be further modified to display at least one probe.

Such probe molecules may be specific for certain target molecules or structures such as cells. The probe molecule may thus be selected from the group comprising small molecules, peptides, glycopeptides, proteins, glycoproteins, receptors, receptor ligands, antibodies, growth factors, hormones, cytokines, chemokines, cell adhesion molecules (CAMs), integrins, vitamins, nucleic acids and derivatives thereof, lipids and glycans.

A preferred probe molecule can be VCAM-1.

If the microcarrier beads are modified to display probe molecules, the method in accordance with the invention can be used to detect the target molecules and/or structures for which the probe molecules are specific.

Thus, methods in accordance with the invention can be adapted to allow isolation and/or detection of specific cell types such as e.g. leukocytes, stem cells, progenitor cells, tumor cells and fetal cells.

The present invention further relates to a flow cytometry device for detecting and/or isolating at least one cell comprising a noozle of a diameter to allow for passing and detecting of cell-associated microcarries.

Such noozles may have a diameter of at least about 200 µm. Other diameters such as at least about 300 µm, at least about 500 µm or at least about 1mm may also be feasible.

The present invention further relates to a device for detecting and/or isolating at least one cell comprising an electrode being capable of immobilizing at least one polarizable microcarrier bead thereto by way of dielectrophoresis.

The present invention also relates to the use of such devices to detect and/or isolate at least one cell.

The present invention further relates to the use of microcarrier beads described hereinafter for detecting and/or isolating at least one cell.

### FIGURE LEGENDS

- Fig. 1: schematically depicts how a microcarrier bead may be covered by different cell types "A" and "B".
- Fig. 2: schematically depicts a comparison between traditional flow cytometry of single individual cells in a flow stream and detection in accordance with the present invention by detecting cell-associated microcarrier beads.
- Fig. 3: schematically depicts the influence on the degree of association of cells to the microcarrier beads on the detection signal.
- Fig. 4: schematically depicts one approach for using dielectrophoresis for detecting cells by coating microcarrier beads therewith.
- Fig. 5: schematically depicts another embodiment of use of dielectrophoresis for detecting cells by making the cells adhere to microcarrier beads.
- Fig. 6: schematically depicts how leukocytes can be isolated from whole blood samples by using "VCAM-1 coated microcarrier beads".

### DETAILED DESCRIPTION OF THE INVENTION

The present invention is based on the finding that microcarrier beads to which cells associate can be used to detect and/or isolate cells by optical detection methods such as flow cytometry and/or dielectrophoresis.

Before some of the embodiments of the present inventions are described in more detail, the following definitions are introduced.

As used in this specification and in the appended claims, the singular forms of "a" and "an" also include the respective plurals unless the context clearly dictates otherwise. Thus, the term "a probe" can include more than one probe, namely two, three, four, five etc. probes.

The terms "about" or "approximately" in the context of the present invention denotes an interval of accuracy that the person skilled in the art will understand to still ensure the technical effect of the feature in question. The term typically indicates deviation from the indicated numerical value of +/- 10%, and preferably +/- 5%.

As has been mentioned above, the invention relates in one embodiment to a method of detecting and/or isolating at least one cell comprising the steps of
- providing at least one microcarrier bead;
- contacting said at least one microcarrier bead with at least one cell such that said at least one cell associates with said at least one microcarrier bead;
- detecting and/or isolating said cell-associated microcarrier bead.

### Microcarrier beads

Thus, the present invention in one aspect relies on the use of microcarrier beads for isolating and/or detecting cells by optical detection methods including flow cytometry, magnetic detection methods and/or dielectrophoresis (DEP).

The term "microcarrier bead" denotes a structure which has a diameter in the range of approximately 20 µm to about 1000µm. Diameters in the range of approximately 75 µm to approximately 300 µm may be preferred as may be diameters in the range of approximately 100 µm to approximately 200 µm.

In some of the preferred embodiments, the beads will display a diameter in the range of about 120 µm to about 180 µm or about 150 µm.

The beads may have any form that is suitable for the purposes of the present invention. They thus may have a rectangular shape, a triangular shape etc. Beads with a round or a substantially round form can be preferred according to the present invention. Thus, the beads will typically have a round, elliptical or spheroid form.

There are no specific requirements as to the structure of the microcarrier beads or as to the materials from which the microcarrier beads are formed except that the beads must be capable of allowing cells to associate with them, and that the structure and the materials must be compatible with the detection technology that is going to be applied, such as flow cytometry, magnetic detection methods and/or dielectrophoresis.

If the cell associated-microcarrier beads are to be analysed by dielectrophoresis, a further requirement is thus that they are polarizable in an inhomogeneous electrical field to experience a force if dielectrophoresis is performed on the beads.

With respect to the aforementioned requirements, the microcarrier beads may be produced from materials such as polystyrene, polydivnylbenzene and polymethylmethacrylate and biodegradable polymers such as poly-lactides, polyclycolides, polycaprolacton and copolymers thereof.

Such beads are in principle commercially available from e.g. SoloHill Engineering, Inc (Ann Arbor, Michigan, USA).

The beads may be further modified, e.g. coated in order to ensure adherence of probe molecules and/or to allow for association of a certain type of cell (see below). Such coatings include glass, collagen (gelatine), a cationic surface treatment, recombinant proteins such as cell adhesion molecules including vascular cell adhesion molecule 1 (VCAM-1) etc.

As will be set out below, a preferred embodiment of the present invention relates to the use of dielectrophoresis for detecting cell-covered microcarrier beads. In this aspect, the present invention can make use of the different polar characteristics of a microcarrier bead versus e.g. a certain cell type. Given that one can influence the microcarrier beads' polarisable characteristics by selecting the appropriate architecture and/or composition, the person skilled in the art will choose the microcarrier depending on the detection method.

If the cell-covered microcarrier beads are used in a dielectrophoresis approach, it may be preferred to use a microcarrier bead whose polarisable properties change depending on whether the microcarrier bead is covered with cells or not.

The person skilled in the art is familiar with the production of microcarrier beads. Thus, one may obtain these beads from aforementioned commercial sources or produce them according to the protocols being described in e.g. Gu et al (J. Control Release (2004), 96.3:463-472), Li et al (Acta Pharmacol. Sin. (2006) 27.6:754-759), Norrgren et al (Dev. Biol. Stand. (1983), 55:43-51) or Tatard et al (Biomaterials (2005), 26.17:3727-3737). Further information as to the production of microcarrier beads that can be used for detection in e.g. DEP approaches can be found in US 2003/0119057 A1 and US 2003/0015428 A1. Thus, the bead technology to create both e.g. microcarrier beads as well as probe covered-beads is known to the skilled person.

Microcarrier beads can e.g. be synthesized e.g. by controlled emulsification techniques such as submerged ink-jet printing.

If a microcarrier bead is to be produced from a polymer such as polystyrene, a polymer solution in a solvent such as dichloromethane can be ink-jetted into an aqueous phase containing a stabilizer for the monodisperse emulsion that is formed. To this end, one can use e.g. polyvinyl alcohol but also proteins could be used to achieve efficient stabilization. Subsequently polymer beads with a narrow size distribution are formed upon removal of the solvent.

If one intends to incorporate e.g. peptides/proteins or nucleic acids as probes which can be water soluble, a double emulsion technique can be used. To this end, the water-soluble peptides and/or proteins may be first dissolved in an aqueous phase and this aqueous phase is emulsified in the polymer solution. If necessary or desired, additional stabilizers such as e.g. Pluronic can be added. This first emulsion is then ink-jetted into a second aqueous phase and beads are again formed upon removal of the solvent.

As has been set out above, the present invention relies partly on the effect that cells are allowed to adhere to the afore-described microcarrier beads and that these cell-covered microcarrier beads are then used to detect the said cells by flow cytometry and/or dielectrophoresis. Contact between cells and microcarrier beads may be established e.g. already during cell culture. Thus, microcarrier beads may be added to a suspension cell culture and the cells will attach to the surface of the microcarrier beads and subsequently continue to grow on the surface of the microcarrier beads.

The person skilled in the art is well-aware that it may be necessary to provide an attachment matrix for certain cell types to allow their growth on the surface of a microcarrier bead. This may be achieved by e.g. coating the microcarrier beads with collagen or polymeric substances that allow adherence of cells to the surface of a microcarrier bead. Such polymers include collagen, fibronectin, hydrogels (acrylamide and agarose gels), polymers linked to CAMs etc.

By way of example, one may add microcarriers such as those available from SoloHill (*vide supra*) to a cell culture of e.g. mammalian cells. If e.g. a suspension cell culture is gently stirred under appropriate conditions, the cells will start adhering to the microcarriers and will form layers of cells on microcarrier beads. The cell-coated microcarrier beads may then be used in the methods in accordance with the invention.

In the context of the present invention the term "cell" denotes all types of cells which can associate with microcarrier beads as described above. Thus, the term may relate to any type of cell that can be isolated from a biological sample such as bodily fluids including blood, plasma, urine, saliva etc. However, samples may also comprise environmental samples taking from soils, lakes, rivers, plants etc.

Typical cell types which can be analysed by the methods in accordance with the invention include e.g. stem cells, progenitor cells, leukocytes, fibroblasts, tumour cells, sperm cells, fibroblasts, neuronal cells, hepatic cells and fetal cells.

Once cells have started to grow on the surface of microcarrier beads, the microcarrier beads may be isolated by simple purification protocols such as centrifugation, magnetic assisted sorting, fluorescence assisted sorting and filtering as well as by washing steps and may be subsequently analysed by optical detection methods including flow cytometry, magnetic detection methods and/or dielectrophoresis.

As will be set out below, the microcarrier beads may be modified to display probe molecules that have a specific affinity for certain target molecules and structures such as certain cell types.

Depending on these modifications, it is thus possible to functionalise the surface of a microcarrier bead and to allow adherence of e.g. only a certain cell type to a microcarrier bead. This allows selective isolation as well as detection of certain cell types by the methods in accordance with the present invention. Depending on the modifications of the microcarrier bead, as well as the initial cell input composition, one can theoretically distinguish different various "cell-bead" complexes.

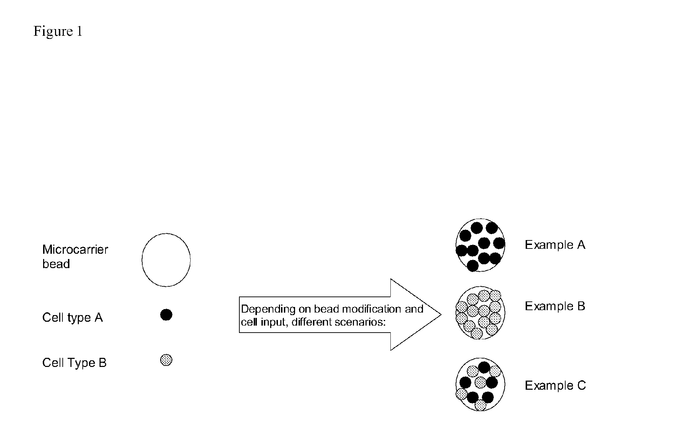

Fig. 1 for example depicts two cellular species in light grey and dark grey that are mixed with a microcarrier bead. If e.g. the surface of a microcarrier is modified with a specific antibody or adhesion molecule, then only a singular cellular species will bind to the specific microcarrier bead. If the adhesion of the cells is non-specific, then different bead-cell complexes containing both cellular species are generated.

Thus, the present invention in one aspect also relates to the use of microcarrier beads in methods in accordance with the invention that display at least one probe molecule. Such a probe molecule may be any type of molecule that is capable of specifically interacting with another target molecule or structure. Thus, probe molecules may be selected from the group comprising small molecules, peptides, glycopeptides, proteins, glycoproteins, receptors, receptor ligands, antibodies, growth factors, hormones, cytokines, chemokines, cell adhesion molecules, integrins, vitamins, nucleic acids and derivatives thereof, lipids and glycans. Typical growth factors include e.g. human growth factor, human nerve growth factor, platelet derived growth factor, TGFβ, EGF, VEGF, TNF-a,TNF-b, FGFs, TGF-a, Epo, IGF-I, IGF-II, IL-1, IL-2, IL-6, IL-8, INF-g and CSFs as well as integrins and CAMs including VLA-1 to VLA-6, LAM-1, Lea-1, CR3, CR4, leukointegrin, collagen, laminin, fibronectin, VCAM-1, MAdCAM-1, E-cadherin, ICAM-1, ICAM-2, ICAM-3, C3b, C4b.

The person skilled in the art will also be aware that fragments of the aforementioned molecules can be used as probe molecules as long as such fragments provide the necessary binding specificity.

A person skilled in the art is also familiar as to how to functionalise e.g. the surface of a microcarrier bead with the aforementioned probe molecules.

Modification of a microcarrier bead with a probe molecule may be achieved by covalent or non-covalent interaction between the microcarrier bead and the probe molecule. To this end, one may e.g. coat the microcarrier bead with a self-assembling polymer. Such a self-assembling polymer may e.g. be polyethylenglycol. The polymers may be further functionalised to introduce chemical functionalities that allow e.g. covalent coupling of an antibody to the surface of the microcarrier bead.

If e.g. antibodies are used as a probe and should be covalently coupled to the microcarrier beads, beads may be used that provide functional groups such as carboxyl groups, amine groups, hydroxyl groups, sulfhydryl groups etc. These groups may then be cross-linked either directly to the antibodies or using a linker that may be homo-or hetero-bifunctional.

Thus, micorcarrier beads may be activated for providing a chemical linkage to the probe molecules by e.g. coating the beads with a polymer having e.g. acyl fluoride functionalities. Other covalent attachment chemistries are also applicable but not limited to anhydrides, epoxides, aldehydes, hydrazides, acyl azides, aryl azides, diazo compounds, benzophenones, carbodiimides, imidoesters, isothiocyanates, NHS esters, CNBr, maleimides, tosylates, tresyl chloride, maleic acid anhydrides and carbonyldiimidazole.

A particular preferred type of probe molecule that can be used to modify a microcarrier bead are molecules that mediates adhesion of a specific cell type. Thus, in a particularly preferred embodiment, the present invention makes use of microcarrier beads, which are modified to display the vascular cell adhesion molecule-1 (VCAM-1). The cell adhesion molecule VCAM-1 is known to specifically bind leukocytes, thus allowing for separation of white blood cells from plasma, platelets and red blood cells in whole blood samples.

Similarly, the microcarrier beads of the present invention may be modified with probe molecules such as antibodies that are specific e.g. for fibroblasts, neuronal cells, hepatic cells, etc.

The person skilled in the art will be able to identify probe molecules that are known to detect e.g. specifically a certain cell type. A person skilled in the art will furthermore be capable of identifying suitable attachment chemistry for either covalently or non-covalently associating such a probe molecule with microcarrier beads.

If such microcarrier beads that have been functionalised additionally with at least one probe molecule are cultivated in the presence of cells, preferably the cell type for which the probe molecule is specific, will adhere to the surface of the microcarrier beads. Flow cytometry and/or dielectrophoresis may then be used to isolate the beads as well as to quantify the number of cells adhered to the beads.

The person skilled in the art is well aware that further elaborations of microcarrier beads can be perceived. Thus, one may e.g. modify the microcarrier beads' surface with e.g. two types of probe molecules and thus select for the presence of two cell types. Similarly, one may use three different types of probe molecules and select for adherence of three different cell types.

Using such an approach it is possible to selectively adhere combinations of cell types to the surface of the microcarrier beads, which e.g. are then capable of e.g. functionally mimicking the interactions in a tumour or normal tissue.

Thus, the methods in accordance with the present invention allow to detect and isolate multi-cellular arrangements as they occur e.g. in tumours by cultivating the cells on functionalised microcarrier beads and subsequent detection of the cells by flow cytometry and/or dielectrophoresis

Before analysis of cell-associated microcarrier beads by optical methods such as flow cytometry and/or dielectrophoresis is discussed in further detail, it is emphasised that the use of microcarriers allows significant acceleration of isolation and detection processes.

As has been explained in the background section, classical flow cytometry relies on the detection of cellular samples comprising single cells. If thus a cellular sample being obtainable from an adherently growing cell culture is to be analysed, the cells must be released from the surface of the e.g. cell culture dishes in which they are grown by e.g. proteologic treatment. This is not necessary for the cell-adhered microcarrier beads that are used in accordance with the present invention.

In addition, typical microcarrier beads as has been set out above are on the order of about 100 to about 200 µm in diameter. A typical white blood cell displays a diameter of about 20 µm. The total surface area of a microcarrier bead is thus in the order of about 30,000 to about 130,000 µm². If one assumes a cell to adhere to a surface on one circular surface that is approximately equal to the diameter of the cell, a typical white blood cell would occupy about 300 µm² of area. Hence, one bead could potentially contain approximately 100 to 400 cells if one assumes that the cells have grown to monolayer coverage of the microcarrier beads. Assuming that the microcarrier beads are counted at a similar speed as in modem flow cytometers (up to 100,000/sec) and if one knows the amount of cells per microcarrier, then the potential throughput of such a method would be on the order of 10 to 40 million cells/sec.

Studies with flow cytometers that operate at very high rates (e.g. about 40,000/per sec) often result in cellular damage and clogging of the instrument. By adhering cells to the microcarrier beads as described above and subsequent analysis by e.g. flow cytometry, it is thus possible to increase the throughput of the system and at the same time to minimise the damage to the cells given that the shear forces for the cell associated-microcarrier beads is reduced compared to the situation where the cells are not grown on the microcarrier beads. Thus, cells which are associated with microcarrier beads are likely more stable and robust for counting in a high flow environment than single cells in suspension.

As has been mentioned above, the cell associated-microcarrier beads may be detected by optical detection methods including flow cytometry, magnetic detection methods and/or dielectrophoresis. While flow cytometry and dielectrophoresis will be discussed in more detail below, the person skilled in the art is aware that optical imaging and image processing approaches may be used as well as microscopic set-ups etc.

### Flow cytometry

Flow cytometry is a technology that is well known to the person skilled in the art in the context of determining the number and type of cells. The person skilled in the art will understand that typical flow cytometers have to be adapted in order to allow determination of the cell associated-microcarrier beads as described above.

Thus, if the cell-adhered microcarrier beads are put in a flow cytometer-type device in accordance with the invention, this instrument should usually have a nozzle larger than at least about 200 µm, assuming a maximum bead diameter of about 150 µm coated with cells of approximately 20 µm diameter. Other feasible values for the nozzle of such a flow cytometer-type device may be larger than at least about 300 µm, at least about 500 µm or at least about 1000 µm.

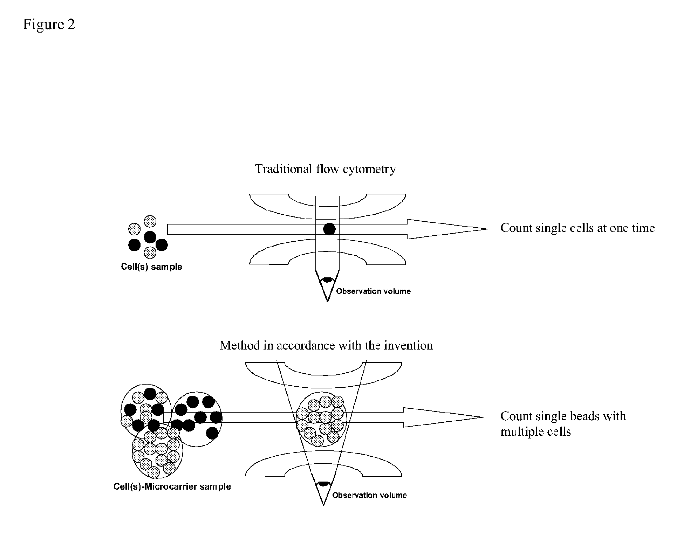

A schematic comparison between traditional flow cytometry and the flow cytometry approaches of the present invention are depicted in Fig. 2. While traditional flow cytometry involves fluidic focussing of single cells through an observation volume, the present invention contemplates detection of cell-adhered microcarrier beads as an alternative to single cell counting. By passing these multi-cell-adhered microcarrier beads through the observation volume, one can simultaneously count multiple cells per bead and in effect thereby multiply the speed at which the overall cell numbers are obtained.

Detection of the cells on the multi-cell adhered microcarrier beads may be performed as in traditional flow cytometry. Thus, one may e.g. use a fluorescent marker that specifically interacts with the cell type to be detected. In one of its more simple embodiments, the methods detect the cell species by fluorescent labelling. This can be achieved by addition of a simple fluorescent and cell-permeable dye, such as Hoechst stain. If multiple cell species are present and require selection, a specific antibody, which target specific surface receptors on the respective cell surfaces and which carry a fluorescent label can be used. By carefully selecting the fluorescent dyes used to label the cells, one can then quantify the cells based on fluorescence intensity.

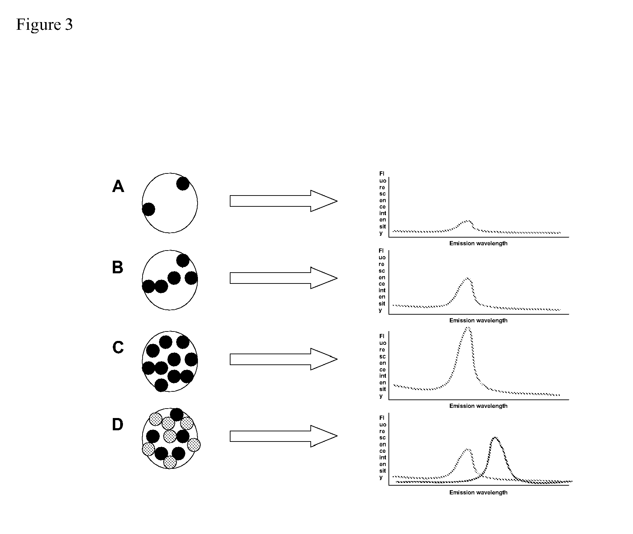

Fig. 3 schematically depicts this approach. Thus, based on fluorescent intensity, the relative number of cells per bead can be obtained. In a scenario, such as Fig. 3 A to C, where only one type of cell is grown on a microcarrier bead at different densities, the fluorescent emission yields a characteristic intensity which is proportional to the number of cells. Furthermore, by use of different fluorescent labels that specifically recognise different types of cells, multi-colour detection allows for quantisation as well as determination of multiple cellular species on a single microcarrier bead (see Fig. 3D).

### Dielectrophoresis

As has been mentioned above, dielectrophoresis can also be used to isolate and detect cells that are grown on microcarrier beads.

Dielectrophoresis is the motion of particles caused by the effects of dielectric polarisation in non-uniform electric fields. Unlike classical electrophoresis where the force acting on a particle is determined by its net charge, dielectrophoresis depends on the volume and dielectric properties of the particle. Thus, when a dielectric particle is exposed to an electric field it polarises. The size and direction of the induced electrical dipole depend on the frequency of the applied field and dielectric properties of the particle and medium such as conductivity, permittivity, morphology and shape of the dielectric particle. Typically, in an inhomogeneous field this causes a force due to the interaction of the induced dipoles and the electric fields.

As mentioned above, the microcarrier beads may be added to cell culture suspensions in order to allow monolayer cell coverage thereof. By maintaining monolayer cell coverage of the microcarrier beads, one can select an upper limit as to how many cells can be bound to a microcarrier bead. As mentioned above, the number of cells depends on the diameter of the microcarrier bead as well as the diameter of the cells to be grown. In a typical scenario, 100 to 400 cells will grow per bead assuming monolayer coverage.

If the coverage of the beads can be controlled, the need for fluorescent-base quantisation of the cells by e.g. flow cytometry can be absolved.

If only a certain type of cellular species is adhered to the microcarrier beads, all beads will display a comparable coverage by this type of cell. Then, each bead would contain an approximately equivalent number of cells and hence only the single beads would require detection in order to produce an overall cell count ((number of beads detected) X (average number of cells per bead) = total cell number). To achieve this, dielectrophoresis (DEP) can be employed in one embodiment of the present invention.

As described above, DEP can move cells by inducing dipole changes within cells upon application of a non-uniform electrical field. Due to the fact that individual cells and microcarrier beads have different size and charge characteristics, they may also possess different DEP characteristics. This means that the electrical field, which may be required to manipulate a bead by DEP may be significantly different from the type of electrical field that one can use to manipulate a single cell. More specifically, the frequency at which negative DEP changes to positive DEP which is also called the "cross-over frequency" can be specific for the type of cell and microcarrier bead depending on the properties chosen for these two components.

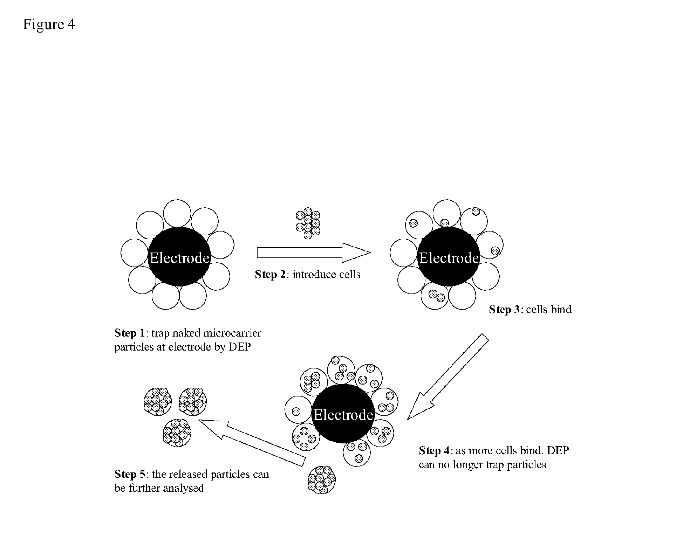

The present invention therefore allows a method for detecting and/or isolating cells in which the electrical field applied ensures that microcarrier beads are trapped at a positive DEP electrode. If such an electrode is immersed into cell culture, the cells may adhere to the microcarrier beads and depending on the increasing intensity by which the microcarrier beads are inhabited by cells, the dielectric properties of the microcarrier beads may change such that a significant alteration of the bead surface properties by e.g. the cell binding, will induce a change in its crossover frequency. This will allow beads, which have been covered with cells to a certain density, to be released from the positive DEP electrode. This aspect of the present invention is schematically depicted in Fig. 4. The cell associated-microcarrier beads can then be analysed e.g. by flow cytometry.

Thus, in one embodiment the present invention relates to a method of detecting and/or isolating cells from a cellular sample in which a microcarrier particle is localised at a DEP electrode and in which coverage of the surface of the microcarrier bead by cells induces release of the cell-associated microcarrier beads from the DEP electrode.

The person skilled in the art will understand that depending on the design of the device that is used for this type of DEP detection, the frequency for trapping the microcarrier beads at the DEP electrode may be manipulated. It may thus be possible to develop an easy-to-perform assay for counting cells in a cellular sample such as a cell culture suspension.

The person skilled in the art is aware that in such an embodiment it is preferred to have microcarrier beads of uniform dimensions given that such a uniform dimension will translate into a uniform frequency requirement for trapping by DEP

This in turn would ensure a uniform coverage of the beads by cells and thus would result in relatively constant "point of release" of the beads from the electrode. The person skilled in the art is clearly aware of further embodiments of this approach by e.g. engineering electrode configurations in multiphase fields within the device that is used for DEP to e.g. levitate microcarrier particles, trapped in a field cage and rotate them or transport them over comparatively long distances.

The person skilled in the art will further be aware that the devices and methods in accordance with the invention may use more than two electrodes such as for example at least two pairs of electrodes as this will allow to achieve the different field strengths more easily. Thus where the figures denote an "electrode", this may mean an electrode pair to create the different field strengths at the desired parts of the device. However, the device may also comprise quadrupole electrodes etc.

Another embodiment of the present invention in which DEP is used to detect and/or isolate cells, relies on the principle described above in reverse order. Thus, microcarrier beads are added to a cell culture suspension and the cells are allowed to adhere to the surface of microcarrier beads. The cell-adhered microcarrier beads are then transferred to a DEP device, for which the DEP frequency of the electrodes has been set to a value to ensure that naked microcarrier beads do not attach to the electrodes. The device can be furthermore tuned in such a way that only microcarrier beads are allowed to attach to the DEP electrode, which are covered by a certain number of cells.

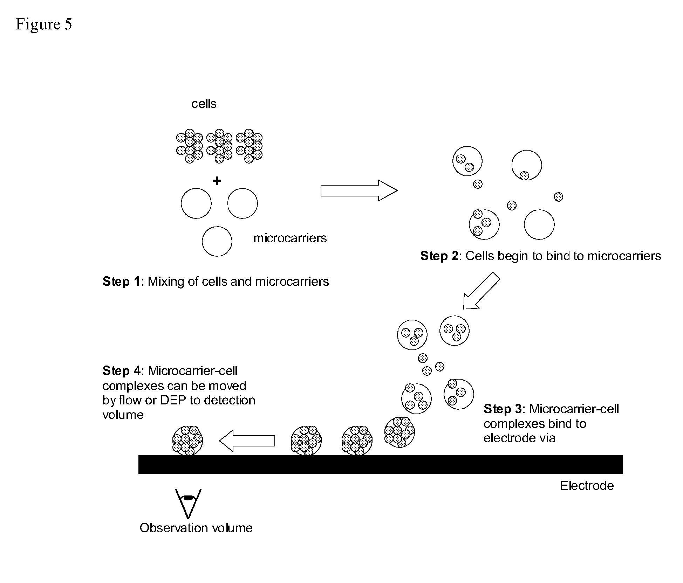

Thus, the cells and "naked" microcarrier beads are first stirred in a suspension in order to allow adherence of the cells to the surface of the microcarrier beads. Once the critical point of cell coverage is achieved, the cell-adhered microcarrier beads become trapped in the DEP field at the DEP electrode and then can be further analysed. A schematic outline of this process is depicted in Fig. 5. In step 1 of Fig. 5, cells are mixed with microcarrier beads. In step 2, cells begin to bind to microcarrier beads. In step 3, a critical point of cell density on the surface of the microcarrier bead is reached which allows the cell-associated microcarrier beads to become attracted to an electrode via DEP. Finally, the DEP electrode fixed cell-associated microcarrier beads are further analysed.

Cells can also be separated or categorised based on their dielectrophoretic response. Thus, one can use dielectrophoresis to isolate microcarrier beads on which cells have been grown. If e.g. the microcarrier beads have been selected to display a movement in an inhomogeneous electric field, one can simply put the cell-associated microcarrier beads in a chamber comprising electrodes for dielectrophoresis and then isolate the beads on which the cells have grown. Such an approach may be particularly interesting if beads are used that are functionalised with probe molecules as one may then separate the cell-associated microcarrier beads from other components of the cellular sample.

It has been set out above that the microcarrier beads of the invention can be functionalised with e.g. probe molecules such as cell adhesion proteins. In the following, one embodiment of the present invention is discussed in which microcarrier beads are functionalised with VCAM-1.

The integrin super family of receptors is in integral component in organising and directing immune cells within the lymphoid systems. In general, integrins are implicated in intercellular adhesion and adhesion to extra cellular matrix components. A broad range of different integrins exists, most of which are expressed on cell surfaces and mediate specific interactions.

The vascular cell adhesion molecule-1 (VCAM-1) is a protein that is induced by inflammatory cytokines, interleucine-1 and tumour necrosis factors on human endothelial cells. VCAM-1 mediates interaction with mononuclear leukocytes via the integrin receptor VLA-4. This interaction ensures adhesion and tethering of rolling lymphocytes to infected parts of the endothelium and thus is of utmost importance to ensure efficient healing of injured and infected sites of the vasculature.

However, one can also use the specific interaction between VCAM-1 and VLA-4 expressing leukocytes in order to isolate such white blood cells from cellular samples such as whole blood.

To this end, one may modify a microcarrier bead with VCAM-1 to create a surface, which is specific for adherence of any type of cells that expresses VLA-4. Typically, such cell types include monocytes and lymphocytes.

For functionalising of microcarrier beads with VCAM-1, one can rely on typical polystyrene beads of approximately 150 µm diameter. VCAM-1 can then be coupled to the surfaces of such beads, which have been functionalised with a reactive group such as e.g. succinimide esters. The succinimide esters will preferably react with primary amines within the VCAM-1 to form a covalent linkage.

If such microcarrier beads are suspended in a mixture with whole blood or other lymphocyte-containing mixtures, this will result in the specific binding of typically only leukocytes to the surface of the functionalised microcarrier surfaces.

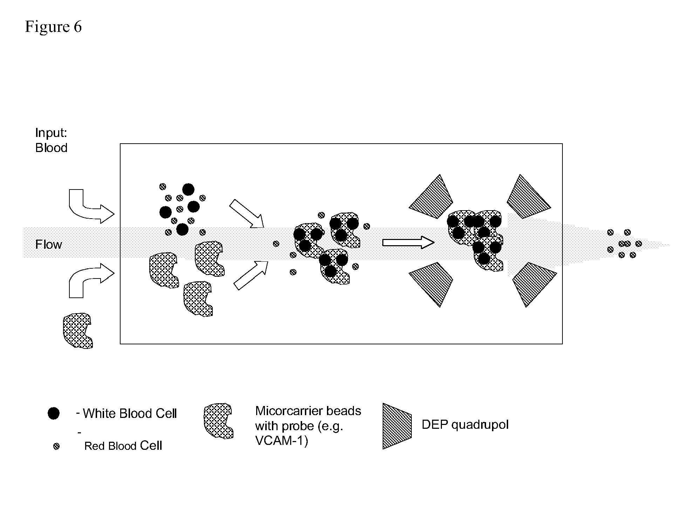

If one now places the microcarrier beads to which the white blood cells have adhered into a non-uniform electrical field, the cells will become polarised and will experience a net driving force due to the non-uniformity of the field. This force can be either in the direction of increasing field strength (so-called "positive DEP") or decreasing field strength (negative DEP). Thus, if one e.g. introduces the VCAM-1 coated-microcarrier beads which have been contacted with e.g. blood, into a DEP quadropole device and applies a fluid to this chamber, one can retain the microcarrier beads on which white blood cells have adhered, within the DEP quadropole while other components, such as red blood cells or plasma proteins are washed away (see Figure 6).

The person skilled in the art is of course also aware that such an approach can be used to isolate other types of cells depending on the nature and the specificity of the probe molecule. If one, for example, coats a microcarrier bead with a probe molecule that is specific for fibroblasts, it may be possible to use DEP to isolate microcarrier beads onto which this cell type has adhered. The same applies for hepatic cells if a corresponding probe molecule is used etc.

The present invention, in essence, relies on the use of microcarrier beads on to which cells have adhered and the detection of such cell-associated microcarrier beads by either flow cytometry and/or dielectrophoresis. In the case of flow cytometry, the use of microcarrier beads has the advantage that the number of cells that can be detected compared to classical approaches is significantly increased. At the same time, the cells are protected against potential harmful factors such as shear forces as they occur during the typical flow cytometric approaches. The use of dielectrophoresis also offers interesting options. Thus, it is for example possible to add microcarrier beads to a cell culture suspension and pass that cell culture suspension continuously through a DEP chamber. Once, cells will have grown on the microcarrier beads to a certain density, the cells will become polarised in the non-uniform electrical field that is applied to the DEP chamber and depending on the specific settings, only cell-associated microcarrier beads of a certain cell density will e.g. be attracted to a specific electrode.

This approach allows cells to be grown on a microcarrier bead to a certain density and to then subsequently isolate such microcarrier beads which may then be further analysed e.g. in flow cytometry.

While the above invention has been described with respect to some of its preferred embodiments, this is in no way to limit the scope of the invention. The person skilled in the art is clearly aware of further embodiments and alterations to the previously described embodiments that are still within the scope of the present invention.

## Claims

1. Method of detecting and/or isolating at least one cell comprising the steps of
- providing at least one microcarrier bead;
- contacting said at least one microcarrier bead with at least one cell such that said at least one cell associates with said at least one microcarrier bead;
- detecting and/or isolating said cell-associated microcarrier bead.

2. Method according to claim 1,
wherein said cell associated-microcarrier bead is detected by optical detection methods including flow cytometry, magnetic detection methods and/or dielectrophoresis.

3. Method according to claim 1 or claim2,
wherein said microcarrier bead has a diameter of about 20 µm to about 1000 µm.

4. Method according to any of claims 1 to 3,
wherein said microcarrier bead comprises bead materials selected from the group comprising polystyrene, polydivnylbenzene and polymethylmethacrylate and biodegradable polymers such as poly-lactides, polyclycolides, polycaprolacton and copolymers thereof.

5. Method according to claim 1,
wherein said contacting step b) occurs in cell culture.

6. Method according to claim 5,
wherein cells form a substantially continuous monolayer on said microcarrier.

7. Method according to any of claims 1 to 6,
wherein said method is used to determine the number of cells in a sample.

8. Method according to any of claims 1 to 7,
wherein said microcarrier is modified to display at least one probe.

9. Method according to claim 9,
wherein said probe is specific for at least one certain cell type.

10. Method according to claim 8 or claim 9,
wherein said probe is selected from the group comprising small molecules, peptides, glycopeptides, proteins, glycoproteins, receptors, receptor ligands, antibodies, growth factors, hormones, cytokines, chemokines, cell adhesion molecules, integrins, vitamins, nucleic acids and derivatives thereof, lipids and glycans.

11. Method according to claim 10,
wherein said probe is VCAM-1 and wherein said method is used to detect leukocytes.

12. Device for detecting and/or isolating at least one cell by flow cytometry comprising a noozle of a diameter to allow for passing and detecting of cell-associated microcarries.

13. Device for detecting and/or isolating at least one cell comprising an electrode setup being capable of immobilizing at least one polarizable microcarrier bead thereto by way of dielectrophoresis.

14. Use of a device according to claim 12 or 13 to detect and/or isolate at least one cell.

15. Use of microcarrier beads which preferably are polarizable for detecting and/or isolating at least one cell.
